# EUROPEAN PATENT SPECIFICATION

(11) **EP 3 387 428 B1**
(45) Date of publication and mention of the grant of the patent: **06.01.2021**
(21) Application number: 16810322.4
(22) Date of filing: 09.12.2016
(51) Int. Cl.: G01N 33/38, G01N 11/10

(54) **DEVICE AND METHOD FOR DETERMINING RHEOLOGICAL PROPERTIES OF CONCRETE**
VORRICHTUNG UND VERFAHREN ZUR BESTIMMUNG RHEOLOGISCHER EIGENSCHAFTEN VON BETON
DISPOSITIF ET PROCÉDÉ DE DÉTERMINATION DE PROPRIÉTÉS RHÉOLOGIQUES DE BÉTON

(30) Priority: 09.12.2015 EP 15198766
(43) Date of publication of application: 17.10.2018
(73) Proprietor: Construction Research & Technology GmbH, 83308 Trostberg (DE)
(72) Inventor: MORO, Sandro, 31057 Silea (IT); MAGAROTTO, Roberta, 31050 Ponzano Veneto (IT); MORATTI, Francesca, 31050 Povegliano (IT)
(86) International application number: PCT/EP2016/080377
(87) International publication number: WO 2017/097954

(56) References cited:
- GB-A- 2 092 308
- JP-A- 55 024 626
- US-A- 5 541 855
- Ray Maker: "Checking out the Kayak Power Meter system", , 7 June 2013 (2013-06-07), XP055658388, Retrieved from the Internet: URL:https://www.dcrainmaker.com/2013/06/ch ecking-power-system.html [retrieved on 2020-01-15]
- HELMER R J N ET AL: "Instrumentation of a kayak paddle to investigate blade/water interactions", PROCEDIA ENGINEERING, vol. 13, 2011, pages 501-506, XP028382562, ISSN: 1877-7058, DOI: 10.1016/J.PROENG.2011.05.121 [retrieved on 2011-07-26]
- Beatriz B. Gomes ET AL: "Paddling Force Profiles at Different Stroke Rates in Elite Sprint Kayaking", JOURNAL OF APPLIED BIOMECHANICS, vol. 31, no. 4, 1 August 2015 (2015-08-01) , pages 258-263, XP055658400, US ISSN: 1065-8483, DOI: 10.1123/jab.2014-0114

## Description

The present invention concerns a device and method using the device for determining rheological properties of concrete.

In the recent past, concrete technology has been pushed towards extreme boundaries: On the one hand, it is nowadays possible to produce members with extreme mechanical and flexural properties, to pour concrete that retains workability for hours under the most severe conditions and to assure very high performances in terms of durability and aesthetics. On the other hand, reaching such extreme boundaries implies working with a material that increasingly becomes difficult to handle, to move, to pump, to pour and to finish.

Additives, such as superplasticizers may be used to improve the rheological properties of the concrete, e.g. of self-compacting concrete. Concrete containing admixtures based on synthetic polymers is commonly denoted as "rheoplastic concrete". Fresh state concrete properties are usually characterized on the jobsite with standardized methods such as slump, slump flow or VEBE tests. The obtained values are used to describe both the initial fluidity of the concrete and its workability retention. However, these methods are not able to characterize the rheological properties of the concrete which may have an important impact on its production. From the mixing time to the surface finishing, through the pumping and placing, the concrete rheology plays a key role on the construction life, but its quantification in each step is not an easy task.

Rheology is, by definition, the study of the flow of matter, primarily in the liquid state, but also as "soft solids" or solids under conditions in which they respond with plastic flow rather than deforming elastically in response to an applied force. As a colloidal system, rheology of concrete can be expressed by the Bingham model. A Bingham fluid is a viscoplastic material that behaves as a rigid body at low stresses but flows as a viscous fluid at high stress. The yield stress (τ₀) determines the value when concrete begins to flow under its own mass. The plastic viscosity (µ) determines the flow time or speed of concrete during pouring or pumping. This value indicates how easily the concrete can be placed or filled into forms.

Rheometers for concrete are designed to characterize the static yield stress, the dynamic yield stress and plastic viscosity of the concrete. A high static yield stress is desirable because it reduces formwork pressure and increases the resistance to segregation. But for ease of pumping, placement, and self-consolidation, a low dynamic yield stress is necessary. The dynamic viscosity provides cohesiveness and contributes to reducing segregation when concrete is flowing. Until now, the tests performed in laboratory and at the job site were focused on evaluating the workability of fresh cementitious materials by measuring change in slump or slump flow. These industrial tests are, in most circumstances, directly correlated to yield stress. Although cementitious materials are not only yield stress fluids, this pragmatic approach was justified by the fact that yield stress is often the most relevant parameter to describe the ability of a material to fill, under its own weight, a formwork or more generally a mold.

Recent trends in modifying mix design (reducing water/binder ratio, adding harsh aggregates, etc.) have shown, however, dramatic consequences on the workability of the material and workers at the building site often complain about these "sticky" concretes that they are unable to place and surface finish. This "stickiness" and, more specifically, the additional stress needed to work the material, is not only related to yield stress but also to plastic viscosity. Therefore, a reduction of both yield stress (τ₀) and plastic viscosity (µ) contributes to improving rheological properties of concrete. The practical evaluation of stickiness and rheology is, in general, a tough task being rather difficult to attribute a single number to the "perceived feeling" of rheology that customers have.

Usually, a rheometer is used to measure yield stress and plastic viscosity values but both these numbers are not so directly and easily correlated to concrete properties measured on the job site and not all job sites are typically equipped with such an instrument.

Therefore, there is a need for improved test methods and devices which are capable of providing meaningful results with respect to some crucial aspects of the work at the job site, like placing, vibration and finishing. Moreover, as these methods and devices should be employed not only in the laboratory but also at the job site, the respective devices should be portable. "Placeability" is used herein as a measure of the ease of moving concrete after pouring.

On a conference held by the European Ready Mixed Concrete Organization ERMCO on June 4 and 5, 2015 in Istanbul (Turkey), the present applicant presented a new portable tool using a pulley system for quantifying the torque needed to move an immersed paddle through concrete. The paddle attached to a movable, wheeled trolley is immersed in a formwork or a bucket or a container filled with concrete. The wheeled trolley is attached to a pulley via a steel wire. The pulley itself is attached to the axis of a rheometer which constantly rotates the pulley with the predetermined rotational speed. The torque measured by the rheometer is concurrently recorded. While this portable device allows for a more realistic determination of concrete properties due to the fact that the movement of a paddle through concrete more closely relates to concrete manipulation done by workers on the job site, this portable device still requires an elaborate setup of different components and an external power source and is therefore mostly used in the laboratory and only of limited use at the job site.

Document US 5,541,855 describes a portable device for testing properties of fresh or unset concrete or mortar. GB 2092308 describes a portable concrete workability meter. Both prior art devices employ rotating probes which are immersed into the concrete or mortar. Accordingly, these devices are essentially portable rotational rheometers and are therefore afflicted with the same problems as conventional laboratory rheometers when it comes to determining rheological properties of concrete: For instance, the rotating probe will cause a radial segregation of the solid particles present in the fluid concrete thus falsifying the measured properties. Moreover, measurements obtained by a rotating probe doe not readily correlate with the subjective "feeling" experienced by a worker on the job site when manually testing concrete properties.

Therefore, the technical problem underlying the present invention can be regarded as providing an improved device and method for determining rheological properties of concrete which can be used on the job site and which does not require any analytical skill of the laborer using the device.

This technical problem is solved by the device and method for determining rheological properties of concrete as defined in the annexed claims.

Accordingly, the present invention concerns a portable, hand-held trowel 1 for determining rheological properties of concrete comprising a handle for manually operating the device, said handle having a front end and a back end, a paddle non-rotatably attached to the front end (13) of said handle, sensor means for determining a force acting on said paddle, an electronic module for evaluating data collected by said sensor means, and an electrical power source for supplying electrical energy to said sensor means and said electronic module.

The present invention provides a compact, self-powered, manually portable trowel which can readily be used at a job site and does not require a sophisticated set-up or elaborate training on part of the user. In order to perform a measurement, the user merely has to immerse the paddle into the concrete and grasp the handle in order to manually move the immersed paddle through the concrete while the force acting on the paddle is recorded. Therefore, the handle exhibits an outer form which can easily be grasped by a hand of the user. Preferably, the handle is configured as an elongated handle bar which has an essentially cylindrical form with a circular, elliptical or otherwise ergonomically convenient cross-section. The paddle can directly be attached to the front end of the handle or indirectly via said sensor means when they are provided at the front end of the handle or between the front end of the handle and the paddle. Preferably, the paddle is attached to the handle via a shaft. When the handle is configured as an essentially cylindrical handle bar, it is preferred the paddle is attached at the front end of the handle bar while the electronic module is provided at the back end of the handle bar in order to counter-balance the weight of the paddle.

As the paddle is non-rotatably attached to the handle, the force acting on the paddle during movement through the concrete can result in a pressure acting on the paddle which can be measured using commercially available pressure sensor or result in a bending force acting on the paddle which can be measured using commercially available force sensors.

In any case, the non-rotatable paddle does not cause radial segregation of solid particles present in the concrete so that the properties measured with the device of the invention are not altered by the measurement process itself. In addition, the device of the present invention mimics a conventional trowel used by workers on the job site so that the measured data can be correlated with the subjective "feeling" experienced by the workers when manually testing concrete properties.

The force data as measured by the trowel can be correlated with rheological properties, respectively properties such as like plastic viscosity.

Preferably, the paddle is removably attached to the handle, allowing the paddle to be changed. In one embodiment, the device comprises at least two exchangeable paddles so that the paddle form and/or surface area can be adapted to various rheological properties of the cementitious materials such as paste, mortar, concrete, etc.

Various sensor means for measuring the force acting on the paddle can be employed in the device of the invention. For instance, force or pressure sensors known in the art can directly be mounted on the paddle. The pressure sensors for measuring a pressure acting on the paddle or the force sensors for measuring bending forces acting on the paddle and/or the peddle shaft preferably employ strain gauges, e.g. resistive strain gauges (metal or semiconductor strain gauges), or piezoelectric sensor.

Due to the linear movement of the paddle during measurement, single-axis strain gauges can be employed. However, in certain embodiments of the invention, multi-axis strain gauges can be used to additionally determine deviations from the linear movement so that, e.g. a warning can be actuated when a measurement might be less reliable due to incorrect handling of the device of the invention.

In a preferred embodiment, the sensor means comprise a sensor head arranged at a front end of the handle. In this case, the paddle is rigidly attached to the sensor head and the force acting on the paddle is converted into a torque acting on the sensor head, e.g.via a shaft connecting the paddle to the sensor head. In a preferred embodiment, the sensor head comprises a flexion leaf on which said paddle is rigidly fixed via the shaft. The bending force acting on the paddle during movement through the concrete results in a torque acting to the flexion leaf which causes a deformation, e.g. a curvature of the flexion leaf. The deformation of the flexion leaf results in strain which can then preferably be measured using a strain gauge as described above. The flexion leaf may comprise a mounting bracket which secures the shaft of the paddle rigidly but releasably to the flexion leaf.

The electronic module for evaluating data collected by said sensor means can comprise an electronic circuit board which is preferably configured to evaluate the maximum force and/or the mean force applied to the paddle through its time of travel. Additionally or alternatively, the electronic module evaluates the integral of the force applied over the distance traveled, i.e. the area under the force-versus-distance or force-versus-time curve. The electronic module may also comprise storage means for storing the measured data and/or an interface for transferring data to an external computer, e.g. a wired interface such as an USB port or a wireless interface such as a Bluetooth or WiFi interface.

Preferably, the electronic module comprises a display. The display have various forms. In one embodiment, the display may comprise a screen, e.g. an LCD or OLED screen. In the harsh environment at the job site, however, a simple and robust display is usually preferred. In one embodiment, the display comprises a digital readout for indicating the evaluated maximum or mean force or the integrated force applied to the paddle. Alternatively or additionally, the display may comprise one or more indicator lights, e.g. one or more light emitting diodes (LEDs). The indicator lights can be used to indicate the status of the device of the invention but also to indicate whether the measured force is within predetermined acceptable boundaries, which may, for instance, be indicated via red and green LEDs, respectively.

According to a preferred embodiment, the sensor means or said electronic module comprises an accelerometer, preferable a 3-axis accelerometer. The accelerometer may be used to determine the deviation of the device from its vertical orientation, specifically the deviation of the paddle from its vertical orientation. As the force acting on the paddle is reduced if the paddle is not oriented perpendicular to the direction of motion, data obtained from the accelerometer can be used to calculate the effective area of the paddle in the direction of motion and to calibrate the measured force data accordingly. In addition or alternatively to multi-axis strain gauges described above, data from the accelerometer can also be used to determine deviations from linear movement of the paddle.

Various electrical power sources can be employed with the device of the present invention. Preferably, an integrated power source is used to allow for a truly independent, portable operation. To this effect, the electrical power source may, for instance comprise a rechargeable or non-rechargeable battery.

The present invention is also directed to a method for determining rheological properties of concrete using a portable, hand-held trowel as defined above. The method according to the invention comprises the steps of
- completely immersing the paddle of said trowel into concrete, thus allowing reproducible results being obtained in subsequent measurements;
- manually moving said paddle along a linear path through the concrete for a predetermined distance;
- measuring a force acting on said paddle during its movement through the concrete and collecting said force data;
- evaluating said measured force data and displaying the evaluated results.

If the paddle is not completely immersed, reproducibility of the results in subsequent measurements is negatively affected. It also preferred that the paddle is kept vertical but, as explained above, deviations from a vertical orientation can automatically be corrected if a device comprising an accelerometer is used.

Preferable, said force data are collected at regular time intervals. As the total distance is predetermined and assuming that the paddle is moved with an essentially constant velocity, the regular time intervals essentially correspond to distance intervals and the area under the force curve represents the energy expended for moving the paddle through the concrete.

According to a preferred embodiment of the method of the invention, said force data are automatically collected when the measured force exceeds a predetermined threshold thus simplifying the operation of the device.

The distance over which the paddle shall be moved through concrete during a measurement is typically selected such that the paddle can sweep the distance without the operator having to change its position. A typical convenient distance is selected between 10 and 60 cm, preferably about 30 cm.

Typically, the evaluated results comprise the maximum force and/or the mean force measured along the path of said paddle and/or an integral over said force data obtained during movement of said paddle. These results can be shown on a display provided on the device. These evaluated results and/or the original data can also be transferred via a wired or wireless data connection to a stationary computer or to a mobile computing device such as a tablet computer or a mobile phone.

The present invention will now be described in more detail referring to a preferred embodiment depicted in the attached drawings.

In the drawings,
- Fig. 1: depicts a perspective view of a portable, hand-held device from measuring rheological properties of concrete according to the invention;
- Fig. 2: shows a side view of the device of figure 1;
- Fig. 3: shows an enlarged view of the force sensor of the device of figures 1 and 2;
- Fig. 4: shows an axial sectional view of the force sensor of figure 3;
- Fig. 5: shows an exemplary force measurement using the device of the invention;
- Fig. 6: shows correlation data of rheological properties of a first type of concrete determined with a conventional test and with the device of the invention, respectively; and
- Fig. 7: shows similar date like Fig. 6 for a second type of concrete.

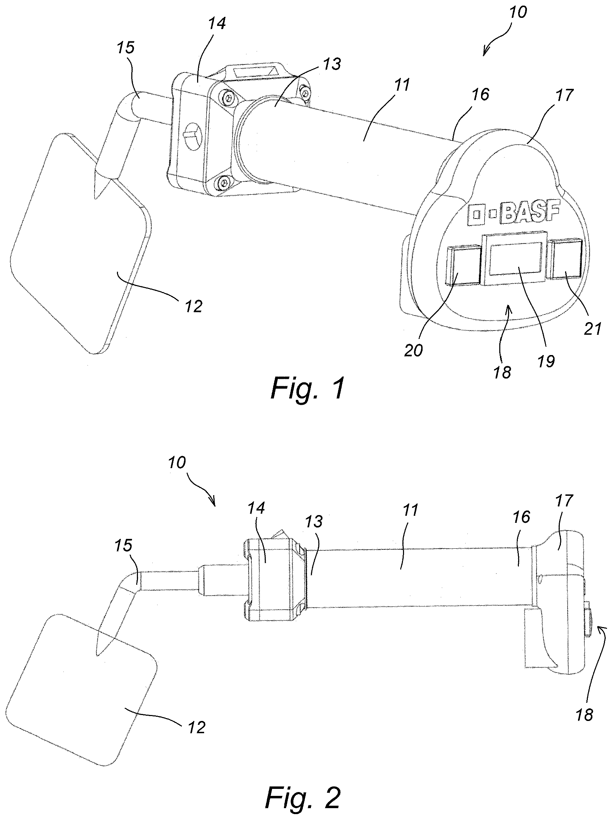

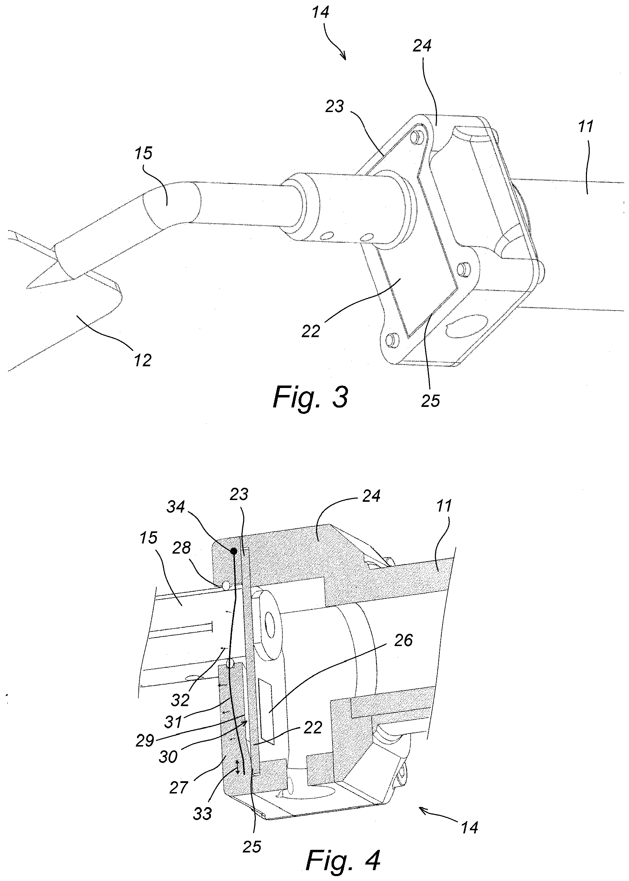

Fig. 1 shows a preferred embodiment of the portable, hand-held device for determining rheological properties of concrete of the invention. The device of the invention has an overall configuration of a hand-held trowel 10. Like a conventional trowel, the trowel 10 of the present invention comprises a handlebar 11 and a paddle 12 attached to the handlebar 11. At its front end 13, the handlebar 11 comprises a sensor head 14 to which the paddle 12 is removable attached via a shaft 15. At its back end 16, the handlebar 11 comprises an electronic module 17 on which a display 18 is provided. In the embodiment depicted in figure 1, the display 18 comprises a digital readout 19 and first and second LED indicator lights 20, 21. The indicator lights 20, 21 can be used as status indicators showing the correct performance of the device. The indicator lights can also be used to show whether the rheological properties of the concrete are within predetermined boundaries, e.g. via red and green lights, respectively. In the sensor head 14, a force sensor is provided, which is capable of measuring the force acting on paddle 12. The force sensor will described in more detail in connection figures 3 and 4 below. In the electronic module 17, an electronic board (not depicted in the drawings) is provided which collects and processes the force data detected by the sensor head 14 and which controls the display 18. The electronic module 17 and/or the handlebar 11 also how houses an electrical power source, which in the present case consists of two AA batteries which may be rechargeable.

Figure 2 shows a side view of the trowel 10 of figure 1.

For determining rheological properties of concrete, the trowel 10 is used as follows: Upon ignition of the trowel 10 via an on/off switch (not depicted in the drawings), the system performs an automatic hardware calibration of the force sensor in order to calibrate the data to ambient temperature. After completion of the initial calibration, paddle 12 is completely immersed into the concrete and the operator manually performs a linear translation of trowel 10 over a predetermined distance, typically 30cm. With start of the movement, system automatically begins to acquire force data acting on the paddle. In a preferred embodiment, the sensor head 14 or the electronic board of the electronic module 17 is also provided with an accelerometer which allows determining the angel of the paddle with respect to its vertical orientation. These data are concurrently collected during movement of the trowel 10. When the translation is finished, the collected data are interpolated and the result of the mean and/or maximum force applied to the paddle is displayed on the digital readout 19 of electronic module 17.

Varied types of force sensors known in the art can be used to determine the force which acts on paddle 12 during its movement thought concrete.

In the preferred embodiment depicted in the drawings, a flexion sensor is used to determine the force acting on the paddle during its movement to the concrete. The flexion sensor translates a force measurement into a torque measurement. This type of measurement will now be described in more detail making reference to figures 3 and 4.

Figure 3 is an enlarged view of the sensor head 14 of trowel 10 of figures 1 and 2. Figure 4 shows a sectional view of sensor head 14.

As can be taken from figure 3, paddle 12 is rigidly connected via shaft 15 to a flexion leaf 22 arranged at the front end of sensor head 14. Flexion leaf 22 has a fixed edge 23 which is fixed at body 24 of the sensor head 14. The opposite edge 25 of flexion leaf 22 is free to move and acts as a floating edge. Consequently, a force acting on the immersed paddle 12 during its movement through the concrete is propagated in a rigid way to flexion leaf 22. Due to its fixed and moveable edges, respectively, the flexion leaf is free to slide and bent in response to a bending force acting on it via shaft 15. The movement of the flexion leaf is measured using a flexion sensor, for instance a strange gauge 26, applied to the bottom side of flexion leaf 22, as can be taken from the sectional view of figure 4. Figure 4 also depicts a front cover 27 which has been omitted in the view of figure 3 for clarity reasons. Cover 27 is provided with an opening 28 through which the shaft 15 can sideably pass. The cover 27 is arranged in a manner so that it does not impede the sliding and bending movement of flexion leaf 22 with the limits necessary to measure the expected forces/torques. To this effect, in the embodiment depicted in the figure 4, the bottom side 29 of cover 27 is provided with a recess 30 allowing flexion leaf 22 to bend upwards in response to the torque applied to leaf 22 by shaft 15. A typical symbolic curvature of flexion leaf 22 in response to the torque applied by shaft 15 is depicted by line 31, with arrows 32 indicating the bending movement and arrow 33 indicating the sliding movement of flexion leaf 22, respectively. Fixed point 34 indicates the fixed end of line 31 (i.e. leaf 22).

In the preferred embodiment depicted in the drawings, the immersed paddle is made of austenitic steel AISI314 while the flexion leaf is made of harmonics steel 38Si7.

Movement of flexion leaf 22 induces a change of electrical resistance in strain gage 26 which is red in a differential way by a Wheatstone bridge provided on the electronic board in electronic module 17. Accordingly, it is possible to compensate for errors induced by environmental temperature and by any electrical resistances in inherent to the system. The output signal of the Wheatstone bridge is passed into a differential amplifier ("rail to rail") thus allowing compensating for errors introduced by variation of the battery voltage. Subsequently, the analog signal is read into a 10-bit resolution analog/digital converter.

In order to compensate changes in the effective area of the paddle when it is not kept vertically during its movement, the data of the 3-axis accelerometer (which can be provided in the sensor head 14 or on the electronic board of the electronic module 17) are evaluated and the force data are appropriately corrected to compensate for changes in the effective area.

A PIC processor collects the digital data and accumulates them as long as a force is applied to the submerged paddle. As soon as the force disappears or falls below a predetermined threshold, data are fitted by the measured angel values of the instrument and the integral of the curve is calculated and the corresponding results are indicated on the display 18. Data maybe shown as digital values on digital readout 19 or even nearly via red and a green LEDs 20, 21, respectively indicated whether the rheological properties of the concrete are within predetermined boundaries or not.

Display 18 usually shows only data of the last measurement. These data can be erased when a new measurement is performed but in accordance with a preferred embodiment, the electronic board also comprises a data storage and/or an interface (e.g. an USB port) allowing data to be transferred to an external computer (e.g. a stationary computer, a tablet computer or a mobile phone) for detailed processing and evaluation. In this case, stored data may not only included summary data such as maximum force or the integral over the force curve of a measurement but may also include all individual measurement points.

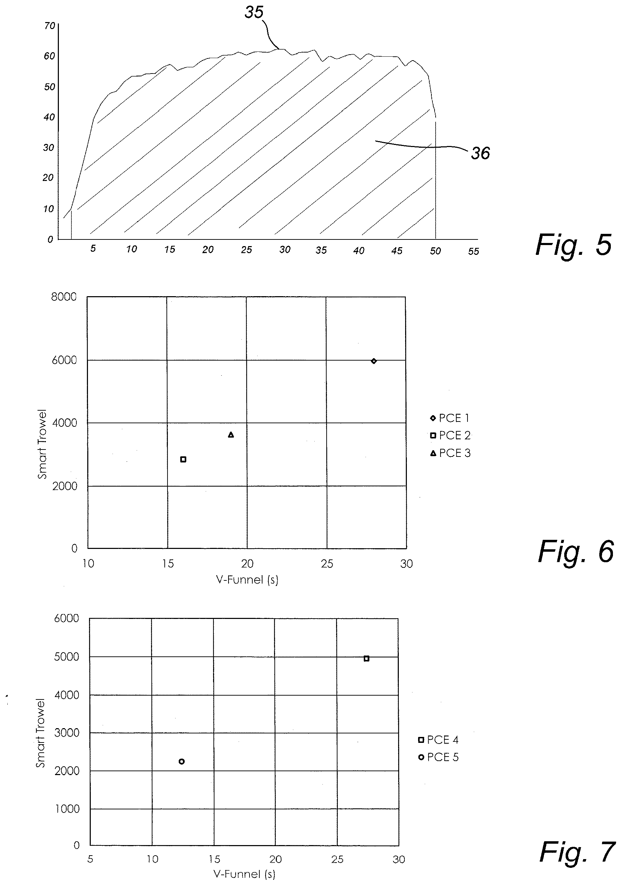

Figure 5 shows the raw data of such a measurement. The immersed paddle is manually moved through concrete over a distance of approximately 30 cm. Data are collected every 0.05 sec. On the x-axis, each data point is sequentially numbered, i.e. the total measurement time of approximately 2.5 sec is shown. The y-axis indicates the resistance of strain gauge 26. The resistance can be calibrated to the force acting on the paddle, i.e. curve 35 depicts a curve which essentially corresponds to the force acting on paddle 12 while the paddle 12 is moved through concrete. The hatched area 36 under curve 35 essentially corresponds to the energy expending while moving the paddle through concrete. Display 18 of the trowel 10 itself only shows the maximum force measured and/or the total area under the force/time curve.

With the devise of the invention, it is possible to detect rheological differences among concretes, e.g. concretes containing different or different amounts of superplasticizers and the measured data correlate which the cohesiveness felt by operators. To show this correlation, the device of the invention has been tested by comparing force data measured with the trowel of figures 1 - 5 with data obtained with a conventional rheological concrete test:

### Examples

In the following examples, the admixtures PCE 1, PCE 3, PCE 4 and PCE 6 are copolymers made from vinyloxybutyl polyethyleneglycol ethoxylate and acrylic acid, while PCE 2 and PCE 5 are polycondensates made from phenoxyethanol polyethyleneglycol ethoxylate, formaldehyde and phenoxyethanol phosphate."

### Example 1:

A first type of concrete was obtained from a mixture of 330 kg/m³ Portland cement (CEM I 45.5 R, Schwenk, Germany), 180 kg/m³ pulverized fuel ash (PFA) with an aggregate mix of 627 kg/m³ sand 0-2 mm, 336 kg/m³ gravel 2-8 mm and 672 kg/m³ gravel 8-16mm. 150 l/m³ of water was added to yield a water/cement ratio of 0.47. Polycarboxylate ether (PCE) superplasticizer obtained from BASF, Germany, was added in three different concentrations, in order to achieve the same fluidity (slump flow measured according to EN 12350-8):
PCE 1: 0.29 % by weight of binder (i.e. cement + PFA)
PCE 2: 0.33 % by weight of binder (i.e. cement + PFA)
PCE 3: 0.65 % by weight of binder (i.e. cement + PFA)

Rheological properties of the concrete were tested with the device of the invention. Three different operators moved the trowel through a basin filled with concrete for a distance of 30 cm and the average force of each measurement was determined. For each PCE concentration, each operator performed three separate measurements and these results were averaged again. For comparison, rheological properties of the concrete were also tested with a classical V-funnel test according to standard EN 12350-9.

Fig. 6 shows correlation data obtained from these measurements with flow time of the V-funnel test shown in seconds on the x-axis and average force date obtained with the device of the invention (denoted "Smart Trowel") in arbitrary units on the y-axis. As can be taken therefrom, the V-funnel data strongly correlate with force data obtained with the device of the invention.

A second type of concrete was obtained from a mixture of 400 kg/m³ Portland cement (CEM I 42.5 R, Schwenk, Germany), 50 kg/m³ limestone powder with an aggregate mix of 202.75 kg/m³ quartz sand 0-0.5 mm, 16.19 kg/m³ quartz sand 0-1 mm, 790.3 kg/m³ sand 0-4 mm, 267.26 kg/m³ gravel 4-8 mm and 486.94 kg/m³ gravel 8-16mm. 168 l/m³ of water was added to yield a water/cement ratio of 0.42. Polycarboxylate ether (PCE) superplasticizer obtained from BASF, Germany was added in two different concentrations:
PCE 4: 0.15 % by weight of cement
PCE 5: 0.32 % by weight of cement

Similar tests as described for the first type of concrete were carried out and the results are shown in Figure 7. Again, the results obtained with the trowel of the invention and the V-funnel results correlate.

### Example 2:

Rheological properties of four different concretes have been measured using the device of the present invention and a portable rotational rheometer (ICAR 3000 distributed by Germann Instruments, Copenhagen, Denmark), respectively. Both the water/cement ratio (w/c) and the superplasticizer type have been modified. Mixed siliceous-limestone crushed aggregates (sand and coarse) were used together with rapid limestone blended cement. The compositions of the four concretes and the results of a slump test according to EN 12350-2 are summarized in Table 1.

**Table 1**

| Kg/m³ | Concrete 1 | Concrete 2 | Concrete 3 | Concrete 4 |
|---|---|---|---|---|
| Sand 0/4 | 970 | 950 | 1010 | 1010 |
| Coarse 8/16 | 855 | 850 | 822 | 822 |
| CEM II/A-LL 42.5 R | 350 | 350 | 350 | 350 |
| Water/Cement | 0.50 | 0.55 | 0.48 | 0.48 |
| Admixture | PCE 2 | PCE 2 | PCE 1 | PCE 6 |
| | (1,0% bwc) | (0,8% bwc) | (0.8% bwc) | (0.8% bwc) |
| EN 12350-2 Slump (mm) | 215 | 220 | 220 | 210 |

As can be taken from Table 1, the different concretes exhibit similar consistencies as far as the slump test is concerned.

Next, three different operators have worked the concrete according the following procedure:
- Step1:: Each operator moved the concrete with the traditional trowel ranking the four concretes according their feeling about the energy spent for working them.
- Step 2:: Each operator measured three time each concrete with the device of invention (Smart Trowel).
- Step 3:: Measurement of rheological properties (yield stress and plastic viscosity) by ICAR 3000 Rheometer

The results of Steps 1, 2 and 3 are summarized in Tables 2, 3 and 4, respectively:

**Table 2**

| Feeling | Concrete 1 | Concrete 2 | Concrete 3 | Concrete 4 |
|---|---|---|---|---|
| Operator 1 | ++ | + | ++++ | +++ |
| Operator 2 | + | ++ | ++++ | +++ |
| Operator 3 | ++ | + | ++++ | +++ |
| **Average** | **++** | **+** | **++++** | **+++** |

| | | | | |
|---|---|---|---|---|
| "+" = very light; "++" = light; "+++" = heavy; "++++" = very heavy | | | | |

As can be taken from Table 2, Concrete 2 is experienced as being the lightest to be worked, while the Concrete 3 is the heaviest to be worked.

**Table 3**

| Smart Trowel | Concrete 1 | Concrete 2 | Concrete 3 | Concrete 4 |
|---|---|---|---|---|
| Operator 1 | 98,1 | 76,9 | 209,5 | 115,7 |
| Operator 2 | 110,2 | 80,6 | 220,4 | 125,3 |
| Operator 3 | 125,4 | 91,6 | 240,1 | 154,6 |
| **Average** | **111,2** | **83,0** | **223,3** | **131,9** |

The results obtained with the device of the present invention (Smart Trowel) correspond to the subjective ranking of the previous evaluation according to Table 2.

**Table 4**

| ICAR 3000 | Concrete 1 | Concrete 2 | Concrete 3 | Concrete 4 |
|---|---|---|---|---|
| Yield Stress (Pa) | 254,1 | 248,7 | 251,3 | 235,7 |
| Plastic Viscosity (Pa*s) | 75,5 | 30,5 | 164,8 | 198,6 |

As can be taken from Table 4, the values of yield stress are almost identical, corresponding to the similar consistency (slump) properties as indicated in Table 1.

Also, using the ICAR rheometer, Concrete 4 is determined being more viscous than Concrete 3. Thus, the plastic viscosity measurement with ICAR is not fully in line with the subjective experience of the worker on the job site.

This example clearly shows that the device of the present invention is more suitable for measurements which correlate with the subjective experience (feeling) of workers on the job site than measurements conducted with portable rotational rheometer.

### Reference signs:

- 10: trowel
- 11: handlebar
- 12: paddle
- 13: front end of handlebar
- 14: sensor head
- 15: removable shaft
- 16: back end of handlebar
- 17: electronic module
- 18: display
- 19: digital readout
- 20: first indicator LED
- 21: second indicator LED
- 22: flexion leaf
- 23: fixed edge of flexion leaf
- 24: body
- 25: floating edge of flexion leaf
- 26: flexion sensor
- 27: cover
- 28: opening
- 29: bottom side of cover
- 30: recess
- 31: exemplary curvature line
- 32: arrows indicating bending movement
- 33: arrow indicating sliding movement
- 34: fixed point
- 35: force curve
- 36: area under curve 35

## Claims

1. Portable hand-held trowel (10) for determining rheological properties of concrete comprising
a handle (11) for manually operating the trowel, said handle having a front end (13) and a back end (16),
a paddle (12) non-rotatably attached to the front end (13) of said handle (11),
sensor means (14) for determining a force acting on said paddle (12),
an electronic module (17) for evaluating data collected by said sensor means (14), and
an electrical power source for supplying electrical energy to said sensor means and said electronic module.

2. Trowel according to claim 1, wherein said paddle (12) is removably attached to the front end (13) of said handle (11).

3. Trowel according to claim 2, comprising at least two exchangeable paddles.

4. Trowel according to one of claims 1 to 3, wherein said sensor means comprise a strain gauge.

5. Trowel according to one of claims 1 to 4, wherein said sensor means (14) comprise a sensor head arranged at said front end (13) of said handle (11).

6. Trowel according to claim 5, wherein said sensor head (14) comprises a flexion leaf (22) on which said paddle (12) is rigidly fixed via a shaft (15).

7. Trowel according to anyone of the preceding claims, wherein said electronic module (17) comprises a display (18).

8. Trowel according to anyone of the preceding claims, wherein said sensor means (14) or said electronic module (17) comprises an accelerometer.

9. Trowel according to anyone of the preceding claims, wherein said electrical power source comprises a rechargeable or non-rechargeable battery.

10. Method for determining rheological properties of concrete using a portable, hand-held trowel according to anyone of claim 1 - 9, comprising the steps of
- completely immersing said paddle (12) of said trowel into concrete;
- manually moving said paddle (12) along a linear path through the concrete for a predetermined distance selected between 10 and 60 cm;
- measuring a force acting on said paddle (12) during its movement through the concrete and collecting said force data;
- evaluating said measured force data and displaying the evaluated results.

11. Method according to claim 10, wherein said force data are collected at regular intervals.

12. Method according to claim 11, wherein said force data are automatically collected when the measured force exceeds a predetermined threshold.

13. Method according to any one of claims 10 to 12, wherein the predetermined distance is selected at about 30 cm.

14. Method according to any one of claims 10 to 13, wherein said evaluated results comprise the maximum force measured along the path of said paddle (12) and/or an integral over said force data obtained during movement of said paddle.

## Patentansprüche

1. Tragbare Handkelle (10) zum Bestimmen rheologischer Eigenschaften von Beton, die Folgendes umfasst:
einen Griff (11) zum manuellen Arbeiten mit der Kelle, wobei der Griff ein vorderes Ende (13) und ein hinteres Ende (16) aufweist,
ein Blatt (12), das nicht drehbar am vorderen Ende (13) des Griffs (11) angebracht ist,
Sensormittel (14) zum Bestimmen einer auf das Blatt (12) wirkenden Kraft,
ein Elektronikmodul (17) zum Auswerten von durch die Sensormittel (14) erfassten Daten und
eine Stromquelle zum Zuführen von elektrischer Energie zu den Sensormitteln und dem Elektronikmodul.

2. Kelle nach Anspruch 1, wobei das Blatt (12) lösbar am vorderen Ende (13) des Griffs (11) angebracht ist.

3. Kelle nach Anspruch 2, die mindestens zwei austauschbare Blätter umfasst.

4. Kelle nach einem der Ansprüche 1 bis 3, wobei die Sensormittel einen Dehnungsmessstreifen umfassen.

5. Kelle nach einem der Ansprüche 1 bis 4, wobei die Sensormittel (14) einen am vorderen Ende (13) des Griffs (11) angeordneten Sensorkopf umfassen.

6. Kelle nach Anspruch 5, wobei der Sensorkopf (14) eine Biegungsplatte (22), auf der das Blatt (12) über einen Schaft (15) starr befestigt ist, umfasst.

7. Kelle nach einem der vorhergehenden Ansprüche, wobei das Elektronikmodul (17) eine Anzeige (18) umfasst.

8. Kelle nach einem der vorhergehenden Ansprüche, wobei die Sensormittel (14) oder das Elektronikmodul (17) einen Beschleunigungsmesser umfassen.

9. Kelle nach einem der vorhergehenden Ansprüche, wobei die Stromquelle eine wiederaufladbare oder nicht wiederaufladbare Batterie umfasst.

10. Verfahren zum Bestimmen rheologischer Eigenschaften von Beton unter Nutzung einer tragbaren Handkelle nach einem der Ansprüche 1-9, das folgende Schritte umfasst:
- vollständiges Eintauchen des Blatts (12) der Kelle in Beton;
- manuelles Bewegen des Blatts (12) entlang einer geradlinigen Bahn durch den Beton über einen vorher bestimmten Abstand, der so ausgewählt wird, dass er zwischen 10 und 60 cm beträgt;
- Messen einer Kraft, die auf das Blatt (12) während dessen Bewegung durch den Beton wirkt, und Erfassen der Kraftdaten;
- Auswerten der gemessenen Kraftdaten und Anzeigen der ausgewerteten Ergebnisse.

11. Verfahren nach Anspruch 10, wobei die Kraftdaten in regelmäßigen Zeitabständen erfasst werden.

12. Verfahren nach Anspruch 11, wobei die Kraftdaten automatisch erfasst werden, wenn die gemessene Kraft einen vorher bestimmten Schwellenwert überschreitet.

13. Verfahren nach einem der Ansprüche 10 bis 12, wobei der vorher bestimmte Abstand so ausgewählt wird, dass er etwa 30 cm beträgt.

14. Verfahren nach einem der Ansprüche 10 bis 13, wobei die ausgewerteten Ergebnisse die maximale Kraft, die entlang der Bahn des Blatts (12) gemessen wird, und/oder ein Integral über die während der Bewegung des Blatts erhaltenen Kraftdaten umfassen.

## Revendications

1. Truelle portable (10) tenue à la main permettant de déterminer des propriétés rhéologiques d'un béton, comprenant :
un manche (11) permettant de manœuvrer manuellement la truelle, ledit manche présentant une extrémité avant (13) et une extrémité arrière (16),
une pale (12) fixée de manière non rotative à l'extrémité avant (13) dudit manche (11),
un moyen capteur (14) permettant de déterminer une force s'exerçant sur ladite pale (12),
un module électronique (17) permettant d'évaluer des données collectées par ledit moyen capteur (14), et
une source d'alimentation électrique permettant de fournir de l'énergie électrique audit moyen capteur et audit module électronique.

2. Truelle selon la revendication 1, dans laquelle ladite pale (12) est fixée de manière amovible à l'extrémité avant (13) dudit manche (11).

3. Truelle selon la revendication 2, comprenant au moins deux pales interchangeables.

4. Truelle selon l'une des revendications 1 à 3, dans laquelle ledit moyen capteur comprend une jauge de contrainte.

5. Truelle selon l'une des revendications 1 à 4, dans laquelle ledit moyen capteur (14) comprend une tête de capteur agencée au niveau de ladite extrémité avant (13) dudit manche (11).

6. Truelle selon la revendication 5, dans laquelle ladite tête de capteur (14) comprend une lame de flexion (22) à laquelle ladite pale (12) est solidarisée par le biais d'un arbre (15).

7. Truelle selon l'une quelconque des revendications précédentes, dans laquelle ledit module électronique (17) comprend un affichage (18).

8. Truelle selon l'une quelconque des revendications précédentes, dans laquelle ledit moyen capteur (14) ou ledit module électronique (17) comprend un accéléromètre.

9. Truelle selon l'une quelconque des revendications précédentes, dans laquelle ladite source d'alimentation électrique comprend une batterie rechargeable ou non rechargeable.

10. Procédé de détermination de propriétés rhéologiques d'un béton à l'aide d'une truelle portable tenue à la main selon l'une quelconque des revendications 1 à 9, comprenant les étapes suivantes
- immersion complète de ladite pale (12) de ladite truelle dans un béton ;
- déplacement manuel de ladite pale (12) suivant une trajectoire linéaire au travers du béton sur une distance prédéterminée sélectionnée entre 10 et 60 cm ;
- mesure d'une force s'exerçant sur ladite pale (12) au cours de son déplacement au travers du béton et collecte de données relatives à ladite force ;
- évaluation des données relatives à ladite force mesurée et affichage des résultats évalués.

11. Procédé selon la revendication 10, dans lequel les données relatives à ladite force sont collectées à intervalles réguliers.

12. Procédé selon la revendication 11, dans lequel les données relatives à ladite force sont collectées automatiquement lorsque la force mesurée dépasse un seuil prédéterminé.

13. Procédé selon l'une quelconque des revendications 10 à 12, dans lequel la distance prédéterminée est sélectionnée à environ 30 cm.

14. Procédé selon l'une quelconque des revendications 10 à 13, dans lequel lesdits résultats évalués comprennent la force maximale mesurée suivant la trajectoire de ladite pale (12) et/ou une intégrale sur les données relatives à ladite force obtenues au cours du déplacement de ladite pale.
